# EUROPÄISCHE PATENTSCHRIFT

(11) **EP 0 918 745 B1**
(45) Veröffentlichungstag und Bekanntmachung des Hinweises auf die Patenterteilung: **28.11.2001**
(21) Anmeldenummer: 97934485.0
(22) Anmeldetag: 18.07.1997
(51) Int. Cl.: C07C 231/08, C07C 233/47

(54) **VERFAHREN ZUR KATALYTISCHEN HERSTELLUNG VON N-ACYLGLYCINDERIVATEN**
CATALYTIC PREPARATION PROCESS OF N-ACYLGLYCINE DERIVATIVES
PROCEDE DE PREPARATION CATALYTIQUE DES DERIVES DE N-ACYLGLYCINE

(30) Priorität: 25.07.1996 DE 19629717
(43) Veröffentlichungstag der Anmeldung: 02.06.1999
(73) Patentinhaber: Aventis Research & Technologies GmbH & Co KG, 65926 Frankfurt am Main (DE)
(72) Erfinder: GEISSLER, Holger, D-55116 Mainz (DE); BOGDANOVIC, Sandra, D-60322 Frankfurt am Main (DE); BELLER, Matthias, 85737 Ismaning (DE); VOLLMÜLLER, Frank, 80333 München (DE); ECKERT, Markus, 80805 München (DE)
(74) Vertreter: Ackermann, Joachim, Dr.
(86) Internationale Anmeldenummer: EP9703853
(87) Internationale Veröffentlichungsnummer: WO9804518

(56) Entgegenhaltungen:
- EP-A- 0 170 830
- EP-A- 0 197 659
- EP-A- 0 338 330
- DE-A- 2 115 985
- US-A- 4 547 590

## Beschreibung

Die vorliegende Erfindung betrifft ein neues, verbessertes Verfahren zur katalytischen Herstellung von N-Acylglycinderivaten durch Umsetzung eines Aldehyds mit einem Carbonsäureamid und Kohlenmonoxid in Gegenwart einer Palladiumverbindung, eines ionischen Halogenides und einer Säure als Katalysator.

Ein derartiges, als Amidocarbonylierung bezeichnetes Verfahren, welches der Reaktionsgleichung 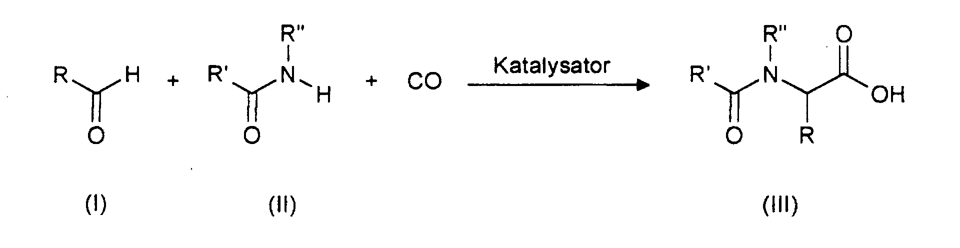 folgt, wurde zuerst von Wakamatsu et al., Chemical Communications 1971, Seite 1540 und in DE-A-2 115 985 beschrieben. Die Umsetzung wurde in Gegenwart von Wasserstoffgas mit einem Mol-Verhältnis CO : H₂ = 3 : 1 durchgeführt. Als Katalysator wurde Cobaltcarbonyl Co₂(CO)₈ in einer Konzentration von 30 mmol Co-Metall je Liter Reaktionsgemisch eingesetzt.

Dasselbe Verfahren, ebenfalls in Gegenwart von Wasserstoffgas und unter zusätzlicher Verwendung einer eine Sulfoxidgruppe enthaltenden Verbindung als Promotor, beschreibt die EP-A-0 170 830. Der Cobaltkatalysator wird dort in einer Konzentration von 100 mmol Co-Metall je Liter Reaktionsgemisch eingesetzt.

Die verhältnismäßig großen Katalysatormengen, die in diesen Verfahren eingesetzt werden, bereiten jedoch erhebliche Schwierigkeiten bei ihrer Abtrennung vom ausreagierten Reaktionsgemisch.

Die EP-B-0 338 330 beschreibt ein Verfahren zur Herstellung von N-Acylglycinderivaten der allgemeinen Formel (III), worin R" Wasserstoff bedeutet, unter Verwendung eines Gemisches aus einer Palladiumverbindung und einem ionischen Halogenid als Katalysator. In dem beschriebenen Verfahren wird die Palladiumverbindung, berechnet auf Palladiummetall, in einer Konzentration von 2 - 10 mmol je Liter Reaktionsgemisch und das ionische Halogenid mit 0,05 - 0,5 mol je Liter Reaktionsgemisch eingesetzt. Die Reaktion wird bei einem Druck von 120 bar und einer Temperatur von 120°C durchgeführt. Die danach erzielte maximale Ausbeute lag bei 89,9%.

In der DE-A-2 115 985 ist ebenfalls die Verwendung eines Palladium enthaltenden Katalysators für die Amidocarbonylierung vorgeschlagen. Danach wird Acetaldehyd und Acetamid in Gegenwart von Palladiumdichlorid und konz. Chlorwasserstoff unter CO/H₂ bei einem Druck von 200 bar und einer Temperatur von 160°C umgesetzt, wobei jedoch die entsprechende N-Acylaminosäure lediglich in einer Ausbeute von etwa 25%, bezogen auf das Acetamid, erhalten wird.

Die hier verwendeten verhältnismäßig hohen Temperaturen und Drücke bereiten jedoch erhebliche Schwierigkeiten bei der Überführung dieser Verfahren in größere Anlagen. Gleichfalls sind sie ökologisch unattraktiv in Bezug auf den Energieverbrauch. Es bestand somit die Nachfrage nach einem wirtschaftlich verbesserten Verfahren, das auch bei geringen Katalysatormengen und relativ niedrigen Drücken und Temperaturen N-Acylglycinderivate in hohen Ausbeuten und Selektivität liefert.

Diese Aufgabe wird gelöst durch ein Verfahren zur Herstellung von N-Acylglycinderivaten der allgemeinen Formel (III) 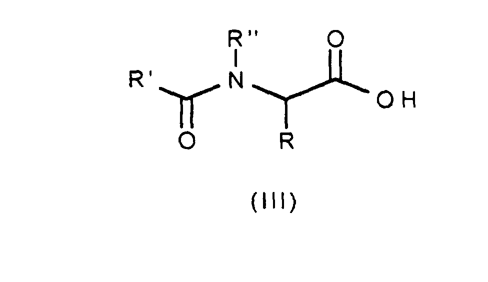 worin
- R: Wasserstoff, eine Carboxylgruppe, ein gesättigter, geradkettiger, verzweigter oder zyklischer (C₁-C₁₀)Alkylrest, ein ein- oder mehrfach ungesättigter, geradkettiger, verzweigter oder zyklischer (C₂-C₁₀)Alkenylrest, ein (C₆-C₁₈)-Arylrest, ein (C₆-C₁₈)-Heteroarylrest, ein (C₁-C₁₀)Alkyl-(C₆-C₁₈)Arylrest, ein (C₁-C₁₀)-Alkyl-(C₆-C₁₈)-Heteroarylrest, oder ein gegebenenfalls mehrfach ungesättigter (C₂-C₁₀)Alkenyl-(C₆-C₁₈)-Arylrest, wobei ein oder mehrere Reste -CH₂- durch C=O oder -O- ersetzt sein können,
- R': Wasserstoff, ein gesättigter, geradkettiger, verzweigter oder zyklischer (C₁-C₂₆)Alkylrest, ein ein- oder mehrfach ungesättigter, geradkettiger, verzweigter oder zyklischer (C₂-C₂₄)Alkenylrest, ein (C₆-C₁₈)Arylrest, ein (C₁-C₁₀)Alkyl-(C₆-C₁₈)Arylrest oder ein gegebenenfalls mehrfach ungesättigter (C₂-C₁₀)Alkenyl-(C₆-C₁₈)Arylrest
und
- R": Wasserstoff, ein gesättigter, geradkettiger, verzweigter oder zyklischer (C₁-C₂₆)Alkylrest, ein ein- oder mehrfach ungesättigter, geradkettiger, verzweigter oder zyklischer (C₂-C₂₃)Alkenylrest, ein (C₆-C₁₈)Arylrest, ein (C₁-C₁₀)Alkyl-(C₆-C₁₈)-Arylrest oder ein gegebenenfalls mehrfach ungesättiger (C₂-C₁₀)Alkenyl-(C₆-C₁₈)Arylrest bedeuten,
wobei man ein Carbonsäureamid der allgemeinen Formel (II) 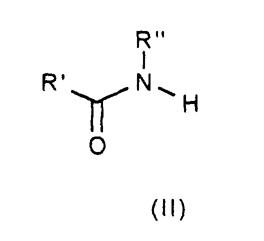 worin R' und R" die oben angegebene Bedeutung besitzen, mit einem Aldehyd der Formel RCHO, worin R die oben angegebene Bedeutung besitzt, in Gegenwart eines Lösungsmittels und eines Gemisches aus einer Palladiumverbindung, einem ionischen Halogenid und einer Säure als Katalysator bei einer Temperatur von 20-200°C und einem CO-Druck von 1-150 bar carbonyliert.

Bevorzugt bedeuten:
- R: Wasserstoff, eine Carboxylgruppe, einen gesättigten, geradkettigen, verzweigten oder zyklischen (C₁-C₆)Alkylrest oder einen ein- oder mehrfach ungesättigten, geradkettigen, verzweigten oder zyklischen (C₂-C₆)Alkenylrest, wobei ein oder mehrere Reste -CH₂- durch C=O oder -O-ersetzt sein können,
- R': einen gesättigten, geradkettigen oder verzweigten (C₈-C₂₄)Alkylrest, insbesondere (C₁₀-C₁₈)Alkylrest, einen ein- oder mehrfach ungesättigten, geradkettigen oder verzweigten (C₈-C₂₄)Alkenylrest, insbesondere (C₁₀-C₁₈)Alkenylrest
und
- R": Wasserstoff, einen gesättigten, geradkettigen oder verzweigten (C₁-C₁₂)Alkylrest, insbesondere (C₁-C₄)Alkylrest oder einen ein- oder mehrfach ungesättigten, geradkettigen oder verzweigten (C₂-C₈)Alkenylrest.

Die Reste R, R' und R" können gegebenenfalls substituiert sein. Beispiele für geeignete Substituenten sind die Hydroxylgruppe, (C₁-C₁₀)-Alkoxyreste, (C₁-C₁₀)-Thioalkoxyreste, Di-(C₁-C₁₈)-alkylaminogruppen, (C₁-C₁₈)-Alkylaminogruppen, Aminogruppen, geschützte Aminogruppen (mit Boc, Z-, Fmoc etc.), Nitrogruppen, (C₁-C₁₀)-Acyloxyreste, Chlorid, Bromid, Cyanid oder Fluor.

Erfindungsgemäß können als Ausgangsamide beliebige Säureamide verwendet werden. Beispiele für geeignete Amide sind Formamid, Acetamid, N-Methylacetamid, N-lsobutylacetamid, Benzamid, Phenylessigsäureamid, N-Butylacetamid, Propionamid, Butyramid, Acrylamid, N-Methylformamid, N-Methylbenzamid, Benzamid und Crotonamid.

Bevorzugte Ausgangsamide für das erfindungsgemäße Verfahren sind Amide und N-Alkylamide, insbesondere N-Methylamide, der geradkettigen oder verzweigten, gesättigten oder ungesättigten Carbonsäuren mit 8 bis 24 C-Atomen, wie z.B Octansäureamid, 2-Ethylhexansäureamid, Decansäureamid, Laurinsäureamid, Palmitinsäureamid, Stearinsäureamid, Ölsäureamid, Linolsäureamid, Linolensäureamid, Gadoleinsäureamid und Nervonsäureamid.

Hiervon sind besonders bevorzugte Beispiele die N-Methylamide von natürlichen Fettsäuren, wie Laurinsäure, Palmitinsäure, Stearinsäure und Ölsäure.

Die Amide der Formel (II) können als Reinsubstanzen oder als Gemische eingesetzt werden. Geeignete Gemische stellen die natürlich vorkommenden Fette dar, z.B. Kokosnuß-, Babassu-, Palmkern-, Oliven-, Ricinus-, Erdnuß-, Raps-, Rinder-, Schweine-, Walfischfett bzw. -Öl (zur Zusammensetzung dieser Fette s. Fieser und Fieser, Organische Chemie, Verlag Chemie 1972, Seite 1208).

Für das erfindungsgemäße Verfahren können beliebige Aldehyde eingesetzt werden. Beispiele für geeignete Aldehyde RCHO, worin R die vorstehend angegebene Definition hat, sind Formaldehyd, Acetaldehyd, Propionaldehyd, Butyraldehyd, Isobutyraldehyd, Furfural, Crotonaldehyd, Acrolein, Benzaldehyd, Phenylacetaldehyd, 2,4-Dihydroxyphenylacetaldehyd, Glyoxalsäure und α-Acetoxypropionaldehyd. Es können auch Dialdehydverbindungen eingesetzt werden. Ebenfalls geeignet sind Substanzen, welche unter den genannten Reaktionsbedingungen einen Aldehyd bilden können, z.B. Aldehydoligomere, wie Paraformaldehyd und Paraldehyd. Es hat sich in vielen Fällen bewährt, Formaldehyd in Form von Paraformaldehyd einzusetzen.

Der Aldehyd wird zweckmäßigerweise in einer Menge von 70 bis 200 mol%, bevorzugt 100 bis 150 mol%, bezogen auf das Carbonsäureamid, eingesetzt.

Das erfindungsgemäße Verfahren wird bevorzugt in einer Stufe ausgeführt. Das Carbonsäureamid und der Aldehyd werden dabei in Gegenwart des Katalysators mit Kohlenmonoxid zum Endprodukt umgesetzt. Überraschenderweise wurde gefunden, daß als Katalysator ein Gemisch aus einer Palladiumverbindung, einem ionischen Halogenid und einer Säure besonders wirksam ist, so daß für den Gesamtprozeß Umsätze von 100% des Carbonsäureamids bei Selektivitäten von 98% zum N-Acylaminosäurederivat erreicht werden, d.h. auch die Ausbeuten an Zielprodukt 98% betragen.

Falls erwünscht, kann das Verfahren auch in zwei Stufen durchgeführt werden. In der ersten Stufe wird dabei zunächst aus dem Aldehyd und dem Carbonsäureamid das N-Acylaminomethylol der Formel (IV), gegebenenfalls unter Zusatz einer Säure als Katalysator, gebildet, welches im zweiten Schritt in Gegenwart eines Katalysators mit Kohlenmonoxid zum Endprodukt umgesetzt wird, wobei in der zweiten Stufe das Gemisch aus einer Palladiumverbindung, einem ionischen Halogenid und einer Säure eingesetzt wird. Die als Katalysator zugesetzte Säure der ersten Stufe ist dabei bevorzugt die als Katalysator zugesetzte Säure der zweiten Stufe.

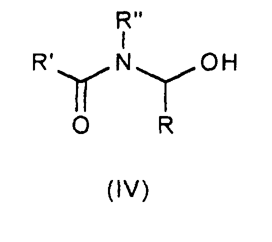

Als Palladiumverbindung können Palladium-II-verbindungen, Palladium-O-verbindungen oder Palladiumphosphinkomplexe verwendet werden. Beispiele für Palladium-II-verbindungen sind Palladiumacetate, -halogenide, -nitrite, -nitrate, -carbonate, -ketonate, -acetylacetonate sowie Allylpalladiumverbindungen. Besonders bevorzugte Vertreter sind PdBr₂, PdCl₂ Li₂PdBr₄, Li₂PdCl₄ und Pd(OAc)₂.
Beispiele für Palladium-0-verbindungen sind Palladiumphosphinkomplexe und Palladiumolefinkomplexe. Besonders bevorzugte Vertreter sind Palladiumbenzylidenkomplexe und Pd(PPh₃)₄.

Beim Einsatz von Palladiumphosphinkomplexen haben sich zudem besonders Bisphosphin-Palladium(II)-verbindungen bewährt. Die Komplexe können als solche eingesetzt werden oder in der Reaktionsmischung aus einer Palladium-II-verbindung, wie z.B. PdBr₂, PdCl₂ oder Palladium-II-acetat unter Zusatz von Phosphinen, wie z.B. Triphenylphosphin, Tritolylphosphin, Bis-(diphenylphosphino)-ethan, 1,4- Bis-(diphenylphosphino)-butan oder 1,3- Bis-(diphenylphosphino)-propan, erzeugt werden.

Durch die Verwendung von Phosphinen mit einem oder mehreren Chiralitätszentren ist es möglich, in der Reaktion zu enantiomerenreinen oder mit einem Enantiomer angereicherten Produkten zu gelangen.

Von diesen Palladiumphosphinkomplexen sind Bis-triphenylphosphin-Palladium(II)-bromid - PdBr₂[PPh₃]₂ - und das entsprechende Chlorid besonders bevorzugt. Diese Komplexe können als solche eingesetzt werden, oder in der Reaktionsmischung aus Palladium-II-bromid bzw. -chlorid und Triphenylphosphin erzeugt werden.

Die Menge an eingesetzter Palladiumverbindung ist nicht besonders kritisch. Aus ökologischen Gründen sollte sie jedoch so gering wie möglich gehalten werden. Für das erfindungsgemäße Verfahren hat sich gezeigt, daß eine Menge von 0,0001 bis 5 mol% Palladiumverbindung (berechnet auf Palladiummetall), insbesondere von 0,001 - 4 mol% und besonders von 0,05 - 2 mol%, bezogen auf das Carbonsäureamid, ausreichend ist.

Als ionisches Halogenid können z.B. Phosphoniumbromide und Phosphoniumiodide, z.B. Tetrabutylphosphoniumbromid bzw. Tetrabutylphosphoniumiodid, sowie Ammonium-, Lithium-, Natrium-, Kaliumbromid und -iodid verwendet werden. Bevorzugte Halogenide sind die Bromide. Vorzugsweise wird das ionische Halogenid in einer Menge von 1 bis 50 mol%, insbesondere von 2 - 40 mol% und ganz besonders von 5 - 30 mol%, bezogen auf das Carbonsäureamid, eingesetzt.

Als Säuren können organische und anorganische Verbindungen mit einem pKₐ<5 (relativ zu Wasser) verwendet werden. So können neben organischen Säuren, wie p-Toluolsulfonsäure, Hexafluorpropansäure oder Trifluoressigsäure und anorganischen Säuren, wie Schwefelsäure oder Phosphorsäure, auch lonenaustauscherharze, wie Amberlyst oder Nafion, verwendet werden.
Von diesen ist Schwefelsäure besonders bevorzugt.
Zweckmäßigerweise wird die Säure in einer Menge von 0,1 bis 20 mol%, insbesondere von 0,2 - 10 mol% und ganz besonders von 0,5 - 5 mol%, bezogen auf das Carbonsäureamid, eingesetzt.

Als Lösungsmittel sind dipolar aprotische Verbindungen bevorzugt einsetzbar. Beispiele dafür sind Dioxan, Tetrahydrofuran, N-Methylpyrrolidon, Ethylenglykoldimethylether, Essigsäureethylester, Essigsäure, Acetonitril, tert. Butylmethylether, Dibutylether, Sulfolan oder N,N-Dimethylacetamid oder Gemische davon. Die Lösungsmittel können in reiner Form oder produktenthaltend bzw. mit Produkt gesättigt eingesetzt werden.

Die aus der Reaktion erhaltenden N-Acyl-α-aminosäuren können in die optisch reinen Aminosäuren überführt werden. Zur stereoselektiven enzymatischen Hydrolyse werden die erhaltenen, racemischen N-Acyl-α-Aminocarbonsäuren üblicherweise in einem wäßrigen Reaktionsmedium gelöst und mit Aminoacylasen, anderen Acylasen bzw. Amidasen oder Carboxypeptidasen versetzt (Lit.: Enzyme Catalysis in Organic Synthesis Ed.: K. Drauz, H. Waldmann, VCH, 1995, Vol. 1, S. 393 ff; J.P. Greenstein, M. Winitz, Chemistry of the Amino Acids; Willey, New York, 1961, Vol. 2, pp 1753). Je nach Spezifität des verwendeten Enzyms erhält man nach Ablauf der Reaktion entweder die ungeschützte (L)-Aminosäure und die (D)-N-Acyl-aminosäure oder man erhält die (D)-Aminosäure und die (L)-N-Acylaminosäure. Die optisch reinen N-Acyl-aminosäuren kann man nach bekannten Methoden entweder in die optisch reinen Aminosäuren überführen, z.B. durch Umsetzung mit Salzsäure, oder in die wiederverwendbaren racemischen N-Acyl-α-Aminocarbonsäuren zurückführen, z. B. mit Acetanhydrid/Eisessig oder durch Zugabe einer Racemase (Takeda Chemical Industries, EP- A-0 304 021; 1989).

Die Reaktion wird im allgemeinen bei Drücken von 1 bis 150 bar, vorzugsweise von 20 bis 100 bar, und bei Temperaturen von 20 bis 200°C, vorzugsweise von 50 bis 150°C, durchgeführt.

Neben den bereits genannten Vorteilen, wie hohe Ausbeute und Selektivität und einfacher technischer Durchführbarkeit, hat das erfindungsgemäße Verfahren den weiteren Vorteil, daß kein Wasserstoffzusatz benötigt wird.

Die folgenden Beispiele sollen das Verfahren erläutern, ohne es darauf zu beschränken.

### Beispiele

### Beispiel 1: (Vergleichsbeispiel)

2,2 g Isovaleraldehyd, 1,5 g Acetamid, 25 ml N-Methylpyrrolidon, 0,092 g Bis(triphenylphosphin)-palladium(II)-chlorid und 0,76 g Lithiumbromid werden in einem 300-ml-Autoklaven bei 120 bar und 120°C umgesetzt. Nach einer Reaktionszeit von 60 Minuten wird das Gemisch mittels Hochdruckflüssigkeitschromatographie (HPLC) analysiert. Man findet 3,9 g N-Acetyl-leucin; dies entspricht einer Ausbeute von 89 %.

### Beispiel 2:

2,2 g Isovaleraldehyd, 1,5 g Acetamid, 25 ml N-Methylpyrrolidon, 0,017 g Palladium(II)-bromid, 0,033 g Triphenylphosphan, 0,025 g Schwefelsäure und 0,76 g Lithiumbromid werden in einem 300-ml-Autoklaven bei 60 bar und 120°C umgesetzt. Nach einer Reaktionszeit von 60 Minuten wird das Gemisch mittels Hochdruckflüssigkeitschromatographie (HPLC) analysiert. Man findet 4,1 g N-Acetyl-leucin; dies entspricht einer Ausbeute von 94 %.

### Beispiel 3: (Vergleichsbeispiel)

2,2 g Isovaleraldehyd, 1,5 g Acetamid, 25 ml N-Methylpyrrolidon, 0,017 g Palladium(II)-bromid, 0,033 g Triphenylphosphan und 0,76 g Lithiumbromid werden in einem 300-ml-Autoklaven bei 60 bar und 80°C umgesetzt. Nach einer Reaktionszeit von 12 Stunden wird das Gemisch mittels Hochdruckflüssigkeitschromatographie (HPLC) analysiert. Man findet 2,4 g N-Acetyl-leucin; dies entspricht einer Ausbeute von 55,4 %.

### Beispiel 4:

2,2 g Isovaleraldehyd, 1,5 g Acetamid, 25 ml N-Methylpyrrolidon, 0,017 g Palladium(II)-bromid, 0,033 g Triphenylphosphan, 0,76 g Lithiumbromid und 0,025 g Schwefelsäure werden in einem 300-ml-Autoklaven bei 60 bar und 80°C umgesetzt. Nach einer Reaktionszeit von 12 Stunden wird das Gemisch mittels Hochdruckflüssigkeitschromatographie (HPLC) analysiert. Man findet 4,0 g N-Acetyl-leucin; dies entspricht einer Ausbeute von 92,4 %.

### Beispiel 5:

2,2 g Isovaleraldehyd, 1,5 g Acetamid, 25 ml N-Methylpyrrolidon, 0,017 g Palladium(II)-bromid, 0,76 g Lithiumbromid und 0,025 g Schwefelsäure werden in einem 300-ml-Autoklaven bei 60 bar und 80°C umgesetzt. Nach einer Reaktionszeit von 12 Stunden wird das Gemisch mittels Hochdruckflüssigkeitschromatographie (HPLC) analysiert. Man findet 3,9 g N-Acetyl-leucin; dies entspricht einer Ausbeute von 89 %.

### Beispiel 6:

2,2 g Isovaleraldehyd, 1,5 g Acetamid, 25 ml N-Methylpyrrolidon, 0,007 g Palladium(II)-bromid, 0,014 g Triphenylphosphan, 0,025 g Schwefelsäure und 0,76 g Lithiumbromid werden in einem 300-ml-Autoklaven bei 60 bar und 80°C umgesetzt. Nach einer Reaktionszeit von 12 Stunden wird das Gemisch mittels Hochdruckflüssigkeitschromatographie (HPLC) analysiert. Man findet 3,25 g N-Acetyl-leucin; dies entspricht einer Ausbeute von 75,0 %.

### Beispiel 7:

2,2 g Isovaleraldehyd, 1,5 g Acetamid, 25 ml N-Methylpyrrolidon, 0,007 g Palladium(II)-bromid, 0,011 g 1,4-Bis(diphenylphosphino)-butan, 0,025 g Schwefelsäure und 0,76 g Lithiumbromid werden in einem 300-ml-Autoklaven bei 60 bar und 80°C umgesetzt. Nach einer Reaktionszeit von 12 Stunden wird das Gemisch mittels Hochdruckflüssigkeitschromatographie (HPLC) analysiert. Man findet 3,5 g N-Acetyl-leucin; dies entspricht einer Ausbeute von 80,8 %.

### Beispiel 8:

2,2 g Isovaleraldehyd, 1,5 g Acetamid, 25 ml N-Methylpyrrolidon, 0,007 g Palladium(II)-bromid, 0,011 g 1,4-Bis(diphenylphosphino)-butan, 0,025 g Schwefelsäure und 1,31 g Natriumiodid werden in einem 300-ml-Autoklaven bei 60 bar und 80°C umgesetzt. Nach einer Reaktionszeit von 12 Stunden wird das Gemisch mittels Hochdruckflüssigkeitschromatographie (HPLC) analysiert. Man findet 3,6 g N-Acetyl-leucin; dies entspricht einer Ausbeute von 83,1 %.

### Beispiel 9:

2,2 g Isovaleraldehyd, 2,2 g Buttersäureamid, 25 ml N-Methylpyrrolidon, 0,007 g Palladium(ll)-bromid, 0,014 g Triphenylphosphan, 0,025 g Schwefelsäure und 0,76 g Lithiumbromid werden in einem 300-ml-Autoklaven bei 60 bar und 80°C umgesetzt. Nach einer Reaktionszeit von 12 Stunden wird das Gemisch mittels Hochdruckflüssigkeitschromatographie (HPLC) analysiert. Man findet 2,7 g N-Butanoyl-leucin; dies entspricht einer Ausbeute von 53,7 %.

### Beispiel 10:

2,7 g Benzaldehyd, 1,5 g Acetamid, 25 ml N-Methylpyrrolidon, 0,007 g Palladium(II)-bromid, 0,014 g Triphenylphosphan, 0,025 g Schwefelsäure und 0,76 g Lithiumbromid werden in einem 300-ml-Autoklaven bei 60 bar und 80°C umgesetzt. Nach einer Reaktionszeit von 12 Stunden wird das Gemisch mittels Hochdruckflüssigkeitschromatographie (HPLC) analysiert. Man findet 3,2 g N-Acetyl-phenylglycin; dies entspricht einer Ausbeute von 66,2 %.

### Beispiel 11:

2,2 g Isovaleraldehyd, 1,5 g Acetamid, 25 ml Dioxan, 0,007 g Palladium(II)-bromid, 0,014 g Triphenylphosphan, 0,025 g Schwefelsäure und 2,9 g Tetrabutylphosphoniumbromid werden in einem 300-ml-Autoklaven bei 60 bar und 80°C umgesetzt. Nach einer Reaktionszeit von 12 Stunden wird das Gemisch mittels Hochdruckflüssigkeitschromatographie (HPLC) analysiert. Man findet 1,4 g N-Acetyl-leucin; dies entspricht einer Ausbeute von 32,3 %.

### Beispiel 12:

2,2 g Isovaleraldehyd, 1,5 g Benzamid, 25 ml N-Methylpyrrolidon, 0,007 g Palladium(II)-bromid, 0,014 g Triphenylphosphan, 0,025 g Schwefelsäure und 0,76 g Lithiumbromid werden in einem 300-ml-Autoklaven bei 60 bar und 80°C umgesetzt. Nach einer Reaktionszeit von 12 Stunden wird das Gemisch mittels Hochdruckflüssigkeitschromatographie (HPLC) analysiert. Man findet 3,3 g N-Benzoyl-leucin; dies entspricht einer Ausbeute von 56,2 %.

### Beispiel 13:

2,2 g Isovaleratdehyd, 1,5 g Acetamid, 25 ml N-Methylpyrrolidon, 0,017 g Palladium(II)-bromid, 0,033 g Triphenylphosphan, 0,029 g Trifluoressigsäure und 0,76 g Lithiumbromid werden in einem 300-ml-Autoklaven bei 60 bar und 80°C umgesetzt. Nach einer Reaktionszeit von 12 Stunden wird das Gemisch mittels Hochdruckflüssigkeitschromatographie (HPLC) analysiert. Man findet 3,1 g N-Acetyl-leucin; dies entspricht einer Ausbeute von 71,6 %.

### Beispiel 14:

2,2 g Isovaleraldehyd, 1,5 g Acetamid, 25 ml N,N-Dimethylformamid, 0,007 g Palladium(II)-bromid, 0,013 g Triphenylphosphan, 0,025 g Schwefelsäure und 0,76 g Lithiumbromid werden in einem 300-ml-Autoklaven bei 60 bar und 80°C umgesetzt. Nach einer Reaktionszeit von 12 Stunden wird das Gemisch mittels Hochdruckflüssigkeitschromatographie (HPLC) analysiert. Man findet 1,75 g N-Acetyl-leucin; dies entspricht einer Ausbeute von 40,0 %.

### Beispiel 15:

2,2 g Isovaleraldehyd, 1,5 g Acetamid, 25 ml N-Methylpyrrolidon, 0,029 g Tris(dibenzylidenaceton)dipalladium(0), 0,033 g Triphenylphosphan, 0,025 g Schwefelsäure und 0,76 g Lithiumbromid werden in einem 300-ml-Autoklaven bei 60 bar und 80 °C umgesetzt. Nach einer Reaktionszeit von 12 Stunden wird das erhaltene Gemisch mittels Hochdruckflüssigkeitschromatographie (HPLC) analysiert. Man findet 2,62 g N-Acetyl-Leucin; dies entspricht einer Ausbeute von 60 %.

### Allgemeine Arbeitsvorschrift I für die Beispiele 16-20:

Es werden 25,0 ml einer 1M N-Methylpyrrolidon-Lösung des Aldehyds und des Amids mit 16,6 mg Palladium(II)bromid, 33,1 mg Triphenylphosphan, 0,76 g Lithiumbromid und 25 mg Schwefelsäure unter 60 bar Kohlenmonoxiddruck in einem 300-ml-Autoklaven bei 120°C für 12 Stunden umgesetzt. Das Reaktionsgemisch wurde mittels Hochdruckflüssigkeitschromatographie (HPLC) analysiert.

### Beispiel 16:

Nach der allgemeinen Arbeitsvorschrift I wurden 3,1 g para-Fluorbenzaldehyd und 1,5 g Acetamid umgesetzt. Man findet 4,7 g N-Acetyl-para-methoxyphenylglycin; dies entspricht einer Ausbeute von 89 %. Ausgewählte NMR-Daten: ¹H-NMR (400 MHz, DMSO-d₆, 25°C): δ = 8,6 (d, 1H, NH), 5,3 (d, 1H, α-CH), 1,9 (s, 3H, COCH₃).

### Beispiel 17:

Nach der allgemeinen Arbeitsvorschrift I wurden 3,0 g Phenylacetaldehyd und 1,5 g Acetamid umgesetzt. Man findet 2,6 g N-Acetylphenylalanin; dies entspricht einer Ausbeute von 48,3 %. Ausgewählte NMR-Daten: ¹H-NMR (400 MHz, DMSO-d₆, 25°C): δ = 8,2 (d, 1H, NH), 4,4 (dt, 1H, α-CH), 1,8 (s, 3H, COCH₃).

### Beispiel 18:

Nach der allgemeinen Arbeitsvorschrift I wurden 2,6 g 3-Methylthiopropionaldehyd mit 1,5 g Acetamid umgesetzt. Man findet 3,6 g N-Acetylmethionin; dies entspricht einer Ausbeute von 75,3 %. Ausgewählte NMR-Daten: ¹H-NMR( (400 MHz, DMSO-d₆, 25°C): δ = 8,2 (d, 1H, NH), 4,1 (dt, 1H, α-CH), 1,8 (s, 3H, COCH₃).

### Beispiel 19:

Nach der allgemeinen Arbeitsvorschrift I wurden 3,5 g ortho-Chlorbenzaldehyd und 1,5 g Acetamid umgesetzt. Man findet 4,7 g N-Acetyl-ortho-chlorphenylglycin; dies entspricht einer Ausbeute von 82,6 %. Ausgewählte NMRDaten: ¹H-NMR (400 MHz, DMSO-d₆, 25°C): δ = 8,7 (d, 1H, NH), 5,8 (d, 1H, α-CH), 1,9 (s, 3H, COCH₃).

### Beispiel 20:

Nach der allgemeinen Arbeitsvorschrift I wurden 3,9 g 2-Naphthaldehyd und 1,5 g Acetamid umgesetzt. Man findet 4,6 g N-Acetyl-2-Naphthyl-glycin; dies entspricht einer Ausbeute von 75,7 %. Ausgewählte NMR-Daten: ¹H-NMR (400 MHz, DMSO-d₆, 25°C): δ = 8,8 (d, 1H, NH), 5,6 (d, 1H, α-CH), 2,0 (s, 3H, COCH₃).

### Allgemeine Arbeitsvorschrift II für die Beispiele 21-28:

Es werden 25,0 ml einer 1 M N-Methylpyrrolidon-Lösung des Aldehyds und des Amids mit 16,6 mg Palladium(II)bromid, 33,1 mg Triphenylphosphan, 0,76 g Lithiumbromid und 25 mg Schwefelsäure unter 60 bar Kohlenmonoxiddruck in einem 300-ml-Autoklaven bei 120°C für 12 Stunden umgesetzt. Anschließend werden die flüchtigen Bestandteile im Hochvakuum abgetrennt. Der Rückstand wird mit gesättigter wäßriger NaHCO₃-Lösung aufgenommen und mit Chloroform und Essigsäureethylester gewaschen. Die wäßrige Phase wird mit Phosphorsäure auf pH=2 eingestellt und mit Essigsäureethylester extrahiert. Die vereinigten organischen Phasen werden über Magnesiumsulfat getrocknet und das Lösemittel im Vakuum entfernt. Das Produkt wird aus einem geeigneten Lösemittelgemisch umkristallisiert.

### Beispiel 21:

Nach der allgemeinen Arbeitsvorschrift II wurden 2,8 g Cyclohexancarbaldehyd und 1,5 g Acetamid umgesetzt. Man findet 4,9 g N-Acetyl-cyclohexylglycin; dies entspricht einer Ausbeute von 99%. Ausgewählte NMR-Daten: ¹H-NMR (400 MHz, DMSO-d₆, 25°C): δ = 7,9 (d, 1H, NH), 4,1 (dd, 1H, α-CH), 1,8 (s, 3H, COCH₃).

### Beispiel 22:

Nach der allgemeinen Arbeitsvorschrift II wurden 2,2 g Pivalaldehyd und 1,5 g Acetamid umgesetzt. Man findet 4,0 g N-Acetyl-tert.-leucin; dies entspricht einer Ausbeute von 92 %. Ausgewählte NMR-Daten: ¹H-NMR (400 MHz, DMSO-d₆, 25°C): δ = 7,7 (d, 1H, NH), 3,9 (d, 1H, α-CH), 1,8 (s, 3H, COCH₃).

### Beispiel 23:

Nach der allgemeinen Arbeitsvorschrift II wurden 0,8 g Formaldehyd und 3,7 g Phthalimid umgesetzt. Man findet 3,1 g N-Phthaloylglycin; dies entspricht einer Ausbeute von 60 %. Ausgewählte NMR-Daten: ¹H-NMR (400 MHz, DMSO-d₆, 25°C): δ =4,3 (s, 2H, α-CH₂).

### Beispiel 24:

Nach der allgemeinen Arbeitsvorschrift II wurden 2,2 g Isovaleraldehyd und 2,2 g Methoxyacetamid umgesetzt. Man findet 3,0 g N-Methoxyacetyl-leucin; dies entspricht einer Ausbeute von 59%. Ausgewählte NMR-Daten: ¹H-NMR (400 MHz, DMSO-d₆, 25°C): δ = 7,9 (d, 1H, NH), 4,3 (dt, 1H, α-CH), 3,8 (s, 2H, -COCH₂-), 3,3 (s, 3H, -OCH₃).

### Beispiel 25:

Nach der allgemeinen Arbeitsvorschrift II wurden 2,8 g Cyclohexancarbaldehyd und 2,2 g Methoxyacetamid umgesetzt. Man findet 4,9 g N-Methoxyacetylcyclohexylglycin; dies entspricht einer Ausbeute von 85 %. Ausgewählte NMR-Daten: ¹H-NMR (400 MHz, DMSO-d₆, 25°C): δ = 7,6 (d, 1H, NH), 4,2 (dd, 1H, α-CH), 3,9 (s, 2H, -COCH₂-), 3,2 (s, 3H, -OCH₂).

### Beispiel 26:

Nach der allgemeinen Arbeitsvorschrift II wurden 2,2 g Isovaleraldehyd und 3,4 g Phenacetamid umgesetzt. Man findet 5,1 g N-Phenacetyl-leucin; dies entspricht einer Ausbeute von 82 %. Ausgewählte NMR-Daten: ¹H-NMR (400 MHz, DMSO-d₆, 25°C): δ = 8,3 (d, 1H, NH), 4,2 (dt, 1H, α-CH), 3,5 (s, 2H, -COCH₂-).

### Beispiel 27:

Nach der allgemeinen Arbeitsvorschrift II wurden 2,7 g Benzaldehyd und 3,4 g Phenacetamid umgesetzt. Man findet 4,4 g N-Phenacetyl-phenylglycin; dies entspricht einer Ausbeute von 65 %. Ausgewählte NMR-Daten: ¹H-NMR (400 MHz, DMSO-d₆, 25°C): δ = 8,8 (d, 1H, NH), 5,3 (d, 1H, α-CH), 3,6 (s, 2H, -COCH₂-).

### Beispiel 28:

Nach der allgemeinen Arbeitsvorschrift II wurden 2,8 g Cyclohexancarbaldehyd und 1,2 g Formamid umgesetzt. Man findet 1,1 g N-Formyl-cyclohexylglycin; dies entspricht einer Ausbeute von 25 %. Ausgewählte NMR-Daten: ¹H-NMR (400 MHz, DMSO-d₆, 25°C): δ = 8,2 (d, 1H, NH), 7,8 (s, 1H, -CHO), 4,1 (dd, 1H, α-CH).

## Patentansprüche

1. Verfahren zur Herstellung von N-Acylglycinderivaten der allgemeinen Formel (III) 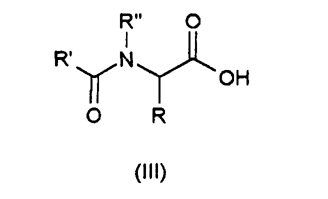 worin
R Wasserstoff, eine Carboxylgruppe, ein gesättigter, geradkettiger, verzweigter oder zyklischer (C₁-C₁₀)Alkylrest, ein ein- oder mehrfach ungesättigter, geradkettiger, verzweigter oder zyklischer (C₂-C₁₀)Alkenylrest, ein (C₆-C₁₈)-Arylrest, ein (C₆-C₁₈)-Heteroarylrest, ein (C₁-C₁₀)-Alkyl-(C₆-C₁₈)Arylrest, ein (C₁-C₁₀)-Alkyl-(C₆-C₁₈)-Heteroarylrest, oder ein gegebenenfalls mehrfach ungesättigter (C₂-C₁₀)Alkenyl-(C₆-C₁₈)-Arylrest, wobei ein oder mehrere Reste -CH₂-durch C=O oder -O- ersetzt sein können,
R' Wasserstoff, ein gesättigter, geradkettiger, verzweigter oder zyklischer (C₁-C₂₆)Alkylrest, ein ein- oder mehrfach ungesättigter, geradkettiger, verzweigter oder zyklischer (C₂-C₂₄)Alkenylrest, ein (C₆-C₁₈)Arylrest, ein (C₁-C₁₀)Alkyl-(C₆-C₁₈)Arylrest oder ein gegebenenfalls mehrfach ungesättigter (C₂-C₁₀)Alkenyl-(C₆-C₁₈)Arylrest
und
R" Wasserstoff, ein gesättigter, geradkettiger, verzweigter oder zyklischer (C₁-C₂₆)Alkylrest, ein ein- oder mehrfach ungesättigter, geradkettiger, verzweigter oder zyklischer (C₂-C₂₃)Alkenylrest, ein (C₆-C₁₈)Arylrest, ein (C₁-C₁₀)Alkyl-(C₆-C₁₈)-Arylrest oder ein gegebenenfalls mehrfach ungesättiger (C₂-C₁₀)Alkenyl-(C₆-C₁₈)Arylrest bedeuten,
wobei R, R' und R" gegebenenfalls substituiert sein können, das **dadurch gekennzeichnet ist, daß** man ein Carbonsäureamid der allgemeinen Formel (II) 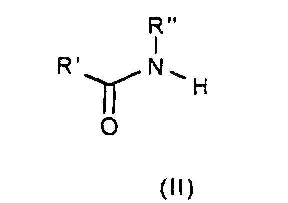 worin R' und R" die oben angegebene Bedeutung besitzen, mit einem Aldehyd der Formel RCHO, worin R die oben angegebene Bedeutung besitzt, in Gegenwart eines Lösungsmittels, einer Palladiumverbindung, einem ionischen Halogenid und einer Säure als Katalysator bei einer Temperatur von 20-200°C und einem CO-Druck von 1-150 bar carbonyliert.

2. Verfahren nach Anspruch 1, **dadurch gekennzeichnet, daß** das Carbonsäureamid der Formel (II) ausgewählt wird unter den Amiden und N-Methylamiden der natürlichen Fettsäuren, Benzamid, Phenylessigsäureamid und 2-Ethylhexanamid.

3. Verfahren nach Anspruch 1, **dadurch gekennzeichnet, daß** R" für Wasserstoff oder (C₁-C₁₂)Alkyl steht.

4. Verfahren nach Anspruch 3, **dadurch gekennzeichnet, daß** R" für Methyl steht.

5. Verfahren nach einem der vorhergehenden Ansprüche, **dadurch gekennzeichnet, daß** die Verbindungen der Formel (II) als Gemische, wie sie aus Naturprodukten erhältlich sind, eingesetzt werden.

6. Verfahren nach einem der vorhergehenden Ansprüche, **dadurch gekennzeichnet, daß** der Aldehyd der Formel (I) ausgewählt wird unter Formaldehyd, Acetaldehyd, Benzaldehyd, Furfural, Propionaldehyd, Butyraldehyd, Glyoxalsäure und Isobutyraldehyd.

7. Verfahren nach einem der vorhergehenden Ansprüche, **dadurch gekennzeichnet, daß** der Aldehyd in Form seines Trimeren oder Oligomeren eingesetzt wird.

8. Verfahren nach Anspruch 7, **dadurch gekennzeichnet, daß** Formaldehyd in Form von Paraformaldehyd eingesetzt wird.

9. Verfahren nach einem der vorhergehenden Ansprüche, **dadurch gekennzeichnet, daß** der Aldehyd in einer Menge von 70 bis 200 Mol-%, bezogen auf das Carbonsäureamid, eingesetzt wird.

10. Verfahren nach einem der vorhergehenden Ansprüche, **dadurch gekennzeichnet, daß** die Palladiumverbindung ausgewählt wird unter Palladium-0-verbindungen, Palladium-II-verbindungen und Palladiumphosphinkomplexen.

11. Verfahren nach Anspruch 10, **dadurch gekennzeichnet, daß** die Palladiumverbindung ausgewählt wird unter PdBr₂, PdCl₂, Pd(OAc)₂ Li₂PdBr₄, Li₂PdCl₄ sowie den Triphenylphosphin-, Tritolylphosphin-, Bis-(diphenylphosphino)ethan-, 1,4-Bis-(diphenylphosphino)butan- und 1,3-Bis-(diphenylphosphino)propankomplexen von Palladium(II).

12. Verfahren nach Anspruch 11, **dadurch gekennzeichnet, daß** als Palladiumverbindung Bis-triphenylphosphin-Palladium(II)-chlorid ( PdCl₂[PPh₃]₂ ), -bromid ( PdBr₂[PPh₃]₂) oder -iodid ( Pdl₂[PPh₃]₂) eingesetzt wird.

13. Verfahren nach Anspruch 10, **dadurch gekennzeichnet, daß** das eingesetzte Phosphin ein oder mehrere Chiralitätszentren enthält.

14. Verfahren nach einem der Ansprüche 10 bis 13, **dadurch gekennzeichnet, daß** die Palladiumverbindung, berechnet auf Palladiummetall, in einer Menge von 0,0001 bis 5 mol% zum Carbonsäureamid eingesetzt wird.

15. Verfahren nach Anspruch 1, **dadurch gekennzeichnet, daß** das ionische Halogenid ausgewählt wird unter Tetrabutylphosphoniumbromid, -iodid, Ammonium-, Lithium-, Natrium- und Kaliumbromid und Ammonium-, Lithium-, Natrium- und Kaliumiodid.

16. Verfahren nach Anspruch 1, **dadurch gekennzeichnet, daß** das ionische Halogenid ein Bromid ist.

17. Verfahren nach Anspruch 1, **dadurch gekennzeichnet, daß** das ionische Halogenid in einer Menge von 1 bis 50 mol% zum Carbonsäureamid eingesetzt wird.

18. Verfahren nach Anspruch 1, **dadurch gekennzeichnet, daß** die Säure eine organische oder anorganische Säure mit einem pKₐ < 5 (relativ zu Wasser) ist.

19. Verfahren nach Anspruch 18, **dadurch gekennzeichnet, daß** die Säure ausgewählt ist unter Schwefelsäure, Trifluoressigsäure, Essigsäure, Hexafluorpropansäure, p-Toluolsulfonsäure, Phosphorsäure und einem lonenaustauscherharz mit einem pKₐ<5 (relativ zu Wasser).

20. Verfahren nach einem der Ansprüche 18 oder 19, **dadurch gekennzeichnet, daß** die Säure in einer Menge von 0,1 bis 20 mol% zum Carbonsäureamid eingesetzt wird.

21. Verfahren nach einem der vorhergehenden Ansprüche, **dadurch gekennzeichnet, daß** das eingesetzte Lösungsmittel Produkt bis zur Sättigungsgrenze enthält.

22. Verfahren nach einem der vorhergehenden Ansprüche, **dadurch gekennzeichnet, daß** die Reaktion bei Drücken von 1 bis 150 bar und bei Temperaturen von 20 bis 200°C durchgeführt wird.

23. Verfahren zur Herstellung von optisch reinen Aminosäuren, **dadurch gekennzeichnet, daß** die racemische N-Acyl-glycinderivate der allgemeinen Formel (III) gemäß dem verfahren nach einem der Ansprüche 1 bis 22 hergestellt werden und anschließend mittels stereoselektiver enzymatischer Hydrolyse zu den entsprechenden optisch reinen Aminosäuren umsetzt.

24. Verfahren nach Anspruch 23, **dadurch gekennzeichnet, daß** die stereoselektive enzymatische Hydrolyse mit einem Enzym ausgewählt unter Acylasen, Amidasen oder Carboxypeptidasen erfolgt.

## Claims

1. A process for preparing N-acylglycine derivatives of the formula (III) 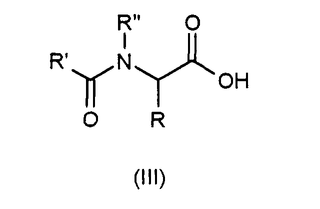 where
R is hydrogen, a carboxyl group, a saturated, straight-chain, branched or cyclic (C₁-C₁₀)alkyl radical, a monounsaturated or polyunsaturated, straight-chain, branched or cyclic (C₂-C₁₀)alkenyl radical, a (C₆-C₁₈)aryl radical, a (C₆-C₁₈)heteroaryl radical, a (C₁-C₁₀) alkyl- (C₆-C₁₈) aryl radical, a (C₁-C₁₀) alkyl-(C₆-C₁₈) heteroaryl radical or a monounsaturated or polyunsaturated (C₂-C₁₀)alkenyl-(C₆-C₁₈)aryl radical, where one or more radicals -CH₂- can be replaced by C=O or -O-,
R' is hydrogen, a saturated, straight-chain, branched or cyclic (C₁-C₂₆)alkyl radical, a monounsaturated or polyunsaturated, straight-chain, branched or cyclic (C₂-C₂₄)alkenyl radical, a (C₆-C₁₈)aryl radical, a (C₁-C₁₀) alkyl- (C₆-C₁₈) aryl radical or a monounsaturated or polyunsaturated (C₂-C₁₀) alkenyl- (C₆-C₁₈) aryl radical
and
R" is hydrogen, a saturated, straight-chain, branched or cyclic (C₁-C₂₆)alkyl radical, a monounsaturated or polyunsaturated, straight-chain, branched or cyclic (C₂-C₂₃)alkenyl radical, a (C₆-C₁₈) aryl radical, a (C₁-C₁₀)alkyl(C₆-C₁₈)aryl radical or a monounsaturated or polyunsaturated (C₂-C₁₀)alkenyl- (C₆-C₁₈)aryl radical,
where R, R' and R" may be substituted, which comprises carbonylating a carboxamide of the formula (II) 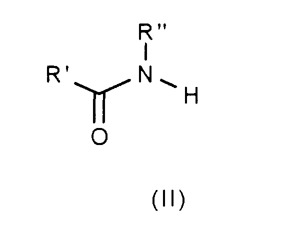 where R' and R" are as defined above, together with an aldehyde of the formula RCHO, where R is as defined above, in the presence of a solvent, a palladium compound, an ionic halide and an acid as catalyst at a temperature of 20-200°C and a CO pressure of 1-150 bar.

2. The process as claimed in claim 1, wherein the carboxamide of the formula (II) is selected from the group consisting of the amides and N-methylamides of natural fatty acids, benzamide, phenylacetamide and 2-ethylhexanoic amide.

3. The process as claimed in claim 1, wherein R" is hydrogen or (C₁-C₁₂)alkyl.

4. The process as claimed in claim 3, wherein R" is methyl.

5. The process as claimed in any of the preceding claims, wherein the compounds of the formula (II) are used as mixtures as are obtainable from natural products.

6. The process as claimed in any of the preceding claims, wherein the aldehyde of the formula (I) is selected from the group consisting of formaldehyde, acetaldehyde, benzaldehyde, furfural, propionaldehyde, butyraldehyde, glyoxalic acid and isobutyraldehyde.

7. The process as claimed in any of the preceding claims, wherein the aldehyde is used in the form of its trimers or oligomers.

8. The process as claimed in claim 7, wherein formaldehyde is used in the form of paraformaldehyde.

9. The process as claimed in any of the preceding claims, wherein the aldehyde is used in an amount of from 70 to 200 mol%, based on the carboxamide.

10. The process as claimed in any of the preceding claims, wherein the palladium compound is selected from the group consisting of palladium(0) compounds, palladium(II) compounds and palladium-phosphine complexes.

11. The process as claimed in claim 10, wherein the palladium compound is selected from the group consisting of PdBr₂, PdCl₂, Pd(OAc)₂, Li₂PdBr₄, Li₂PdCl₄, and also the triphenylphosphine, tritolylphosphine, bis(diphenylphosphino)ethane, 1,4-bis(diphenylphosphino)butane and 1,3-bis(diphenylphosphino)propane complexes of palladium(II).

12. The process as claimed in claim 11, wherein the palladium compound used is bis(triphenylphosphine)palladium(II) chloride (PdCl₂[PPh₃]₂ ), bromide (PdBr₂[PPh₃]₂) or iodide ( PdI₂[PPh₃]₂).

13. The process as claimed in claim 10, wherein the phosphine used contains one or more chiral centers.

14. The process as claimed in any of claims 10 to 13, wherein the palladium compound, calculated as palladium metal, is used in an amount of from 0.0001 to 5 mol% based on the carboxamide.

15. The process as claimed in claim 1, wherein the ionic halide is selected from the group consisting of tetrabutylphosphonium bromide and iodide, ammonium, lithium, sodium and potassium bromide and ammonium, lithium, sodium and potassium iodide.

16. The process as claimed in claim 1, wherein the ionic halide is a bromide.

17. The process as claimed in claim 1, wherein the ionic halide is used in an amount of from 1 to 50 mol% based on the carboxamide.

18. The process as claimed in claim 1, wherein the acid is an organic or inorganic acid having a PKₐ < 5 (relative to water).

19. The process as claimed in claim 18, wherein the acid is selected from the group consisting of sulfuric acid, trifluoroacetic acid, acetic acid, hexafluoropropanoic acid, p-toluenesulfonic acid, phosphoric acid and an ion-exchange resin having a pKₐ<5 (relative to water).

20. The process as claimed in either claim 18 or 19, wherein the acid is used in an amount of from 0.1 to 20 mol% based on the carboxamide.

21. The process as claimed in any of the preceding claims, wherein the solvent used contains product up to the saturation limit.

22. The process as claimed in any of the preceding claims, wherein the reaction is carried out at pressures of from 1 to 150 bar and at temperatures of from 20 to 200°C.

23. A process for preparing optically pure amino acids, which comprises preparing the racemic N-acylglycine derivatives of the formula (III) by the process as claimed in any of claims 1 to 22 and subsequently converting these into the corresponding optically pure amino acids by means of stereoselective enzymatic hydrolysis.

24. The process as claimed in claim 23, wherein the stereoselective enzymatic hydrolysis is carried out using an enzyme selected from the group consisting of acylases, amidases and carboxypeptidases.

## Revendications

1. Procédé pour la préparation de dérivés de N-acylglycine de formule générale (III) 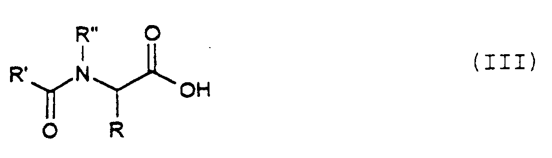 où
R représente un atome d'hydrogène, un groupe carboxyle, un reste alkyle en C₁-C₁₀ saturé, à chaîne droite, ramifiée ou cyclique, un reste alcényle en C₂-C₁₀ une ou plusieurs fois insaturé, à chaîne droite, ramifiée ou cyclique, un reste aryle en C₆-C₁₈, un reste hétéroaryle en C₆-C₁₈, un reste (alkyl en C₁-C₁₀)-aryle en C₆-C₁₈, un reste (alkyl en C₁-C₁₀)-hétéroaryle en C₆-C₁₈, ou un reste (alcényle en C₂-C₁₀)-aryle en C₆-C₁₈ éventuellement plusieurs fois insaturé, un ou plusieurs restes -CH₂- pouvant être remplacés par C=O ou -O-,
R' représente un atome d'hydrogène, un reste alkyle en C₁-C₂₆ saturé, à chaîne droite, ramifiée ou cyclique, un reste alcényle en C₂-C₂₄ une ou plusieurs fois insaturé, à chaîne droite, ramifiée ou cyclique, un reste aryle en C₆-C₁₈, un reste (alkyl en C₁-C₁₀)-aryle en C₆-C₁₈ ou un reste (alcényle en C₂-C₁₀)-aryle en C₆-C₁₈ éventuellement plusieurs fois insaturé,
et
R" représente un atome d'hydrogène, un reste alkyle en C₁-C₂₆ saturé, à chaîne droite, ramifiée ou cyclique, un reste alcényle en C₂-C₂₃ une ou plusieurs fois insaturé, à chaîne droite, ramifiée ou cyclique, un reste aryle en C₆-C₁₈, un reste (alkyl en C₁-C₁₀)-aryle en C₆-C₁₈ ou un reste (alcényle en C₂-C₁₀)-aryle en C₆-C₁₈ éventuellement plusieurs fois insaturé,
où R, R' et R" peuvent éventuellement être substitués, **caractérisé en ce qu'**on carbonyle un amide d'acide carboxylique de formule générale (II) 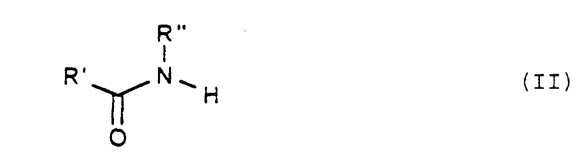 où R' et R" possèdent les significations données ci-dessus, avec un aldéhyde de formule RCHO, où R possède la signification donnée ci-dessus, en présence d'un solvant et d'un mélange d'un composé de palladium, d'un halogénure ionique et d'un acide en tant que catalyseur, à une température de 20 à 200°C et une pression de CO de 1 à 150 bars.

2. Procédé selon la revendication 1, **caractérisé en ce qu'**on choisit l'amide d'acide carboxylique de formule (II) parmi les amides et les N-méthylamides d'acides gras naturels, le benzamide, le phénylacétamide et le 2-éthyl-hexanamide.

3. Procédé selon la revendication 1, **caractérisé en ce que** R" représente un atome d'hydrogène ou un groupe alkyle en C₁-C₁₂.

4. Procédé selon la revendication 3, **caractérisé en ce que** R" représente un groupe méthyle.

5. Procédé selon l'une des revendications précédentes, **caractérisé en ce qu'**on utilise les composés de formule (II) sous forme de mélanges tels que l'on obtient à partir de produits naturel.

6. Procédé selon l'une des revendication précédentes, **caractérisé en ce qu'**on choisit l'aldéhyde de formule (I) parmi le formaldéhyde, l'acétaldéhyde, le benzaldéhyde, le furfural, le propionaldéhyde, le butyraldéhyde, l'acide glyoxalique et l'aldéhyde isobutyrique.

7. Procédé selon l'une des revendications précédentes, **caractérisé en ce qu'**on utilise l'aldéhyde sous forme de ses trimères ou oligomères.

8. Procédé selon la revendication 7, **caractérisé en ce qu'**on utilise le formaldéhyde sous forme de paraformaldéhyde.

9. Procédé selon l'une des revendications précédentes, **caractérisé en ce qu'**on utilise l'aldéhyde en une quantité de 70 à 200 % molaires, par rapport à l'amide d'acide carboxylique.

10. Procédé selon l'une des revendications précédentes, **caractérisé en ce qu'**on choisit le composé de palladium parmi les composés de palladium(0), les composés de palladium(II) et les complexes de palladiumphosphine.

11. Procédé selon la revendication 10, **caractérisé en ce qu'**on choisit le composé de palladium parmi PdBr₂, PdCl₂, Pd(OAc)₂, Li₂PdBr₄, Li₂PdCl₄, ainsi que les complexes de palladium(II) de triphénylphosphine, de tritolylphosphine, de bis-(diphénylphosphino)-éthane, de 1,4-bis-(diphénylphosphino)-butane et de 1,3-bis-(diphénylphosphino)-propane.

12. Procédé selon la revendication 11, **caractérisé en ce qu'**on utilise en tant que composé de palladium, le chlorure de bis-triphénylphosphinopalladium(II) (PdCl₂[PPh₃]₂), le bromure de bis-triphénylphosphino-palladium(II) (PdBr₂[PPh₃]₂) ou l'iodure de bis-triphénylphosphino-palladium(II), (PdI₂[PPh₃]₂).

13. Procédé selon la revendication 10, **caractérisé en ce que** la phosphine utilisée présente un ou plusieurs centres de chiralité.

14. Procédé selon les revendications 10 à 13, **caractérisé en ce qu'**on utilise le composé de palladium, calculé sur le palladium métal, en une quantité de 0,0001 à 5 % molaires par rapport à l'amide d'acide carboxylique.

15. Procédé selon la revendication 1, **caractérisé en ce qu'**on choisit l'halogénure ionique parmi le bromure de tétrabutylphosphonium, l'iodure de tétrabutylphosphonium, les bromures d'ammonium, de lithium, de sodium et de potassium et les iodures d'ammonium, de lithium, de sodium et de potassium.

16. Procédé selon la revendication 1, **caractérisé en ce que** l'halogénure ionique est un bromure.

17. Procédé selon la revendication 1, **caractérisé en ce qu'**on utilise l'halogénure ionique en une quantité de 1 à 50 % molaires par rapport à l'amide d'acide carboxylique.

18. Procédé selon la revendication 1, **caractérisé en ce** l'acide est un acide organique ou inorganique ayant un pKa<5 (par rapport à l'eau).

19. Procédé selon la revendication 18, **caractérisé en ce qu'**on choisit l'acide parmi l'acide sulfurique, l'acide trifluoracétique, l'acide acétique, l'acide hexafluoropropanoïque, l'acide p-toluènesulfonique, l'acide phosphorique et une résine échangeuse d'ions ayant un pKₐ<5 (par rapport à l'eau).

20. Procédé selon l'une des revendications 18 ou 19, **caractérisé en ce qu'**on utilise l'acide en une quantité de 0,1 à 20 % molaires par rapport à l'amide d'acide carboxylique.

21. Procédé selon l'une des revendications précédentes, **caractérisé en ce que** le solvant utilisé contient le produit jusqu'au seuil de saturation.

22. Procédé selon l'une des revendications précédentes, **caractérisé en ce qu'**on met en oeuvre la réaction à une pression de 1 à 150 bars et à une tepérature de 20 à 200°C.

23. Procédé pour la préparation d'aminoacides à pureté optique, **caractérisé en ce qu'**on prépare les dérivés racémiques de N-acyl-glycine de formule générale (III) selon le procédé selon l'une des revendications 1 à 22 et ensuite on fait réagir à l'aide d'une hydrolyse enzymatique stéreosélective pour obtenir les aminoacides optiquement purs correspondants.

24. Procédé selon la revendication 23, **caractérisé en ce qu'**on réalise l'hydrolyse enzymatique stéréosélective à l'aide d'une enzyme prise parmi des acylases, amidases et carboxypeptidases.
